# EUROPÄISCHE PATENTSCHRIFT

(11) **EP 0 812 577 B1**
(45) Veröffentlichungstag und Bekanntmachung des Hinweises auf die Patenterteilung: **13.11.2002**
(21) Anmeldenummer: 97107227.7
(22) Anmeldetag: 30.04.1997
(51) Int. Cl.: A61B 19/00

(54) **Implantierbares Positionier- und Fixiersystem für aktorische und sensorische Implantate**
Implantable positioning and fixation system for actoric and sensoric implants
Système de positionnement et fixation implantable pour implants actoriques et sensoriels

(30) Priorität: 10.05.1996 DE 19618964
(43) Veröffentlichungstag der Anmeldung: 17.12.1997
(73) Patentinhaber: PHONAK AG, 8712 Stäfa (CH)
(72) Erfinder: Lehner, Rolf, Dipl. Ing., 73734 Esslingen (DE); Müller, Gerd, Dr. Dipl. Phys., 85716 Unterschleissheim (DE); Leysieffer, Hans, Dr. Dipl. Ing., 82024 Taufkirchen (DE)
(74) Vertreter: Schwan, Gerhard, Dipl.-Ing.

(56) Entgegenhaltungen:
- US-A- 4 809 694
- US-A- 5 112 336
- US-A- 5 320 628

## Beschreibung

Die Erfindung betrifft eine dauerhaft implantierbare Vorrichtung für die intraoperative Positionierung und nachfolgende Fixierung von implantierbaren aktorischen oder sensorischen Mitteln (im folgenden kurz als Mittel bezeichnet) im menschlichen Körper, insbesondere im Mastoid- und Mittelohrbereich des Schädels.

Angesichts der außerordentlich kleinen und empfindlichen anatomischen Strukturen im menschlichen Körper, insbesondere im Mastoid- und Mittelohrbereich des Schädels, ist das längere (mehr als wenige Sekunden) dauernde, handgeführte Beibehalten der Position eines Mittels nahezu unmöglich oder erfordert vom Operateur einen erheblichen Kraftund Konzentrationsaufwand. Viele Eingriffe im Körper, vor allem im Schädelbereich, erfordern aber gerade eine über längere Zeiträume fixierbare, zielpunktgerichtete Positionierung geeigneter aktorischer bzw. sensorischer Mittel.

Aufgrund der Tatsache, daß handgeführte aktorische oder sensorische Mittel für mikrochirurgische, therapeutische oder diagnostische Manipulationen an empfindlichen Kleinstrukturen z.B. des Schädels stets das Risiko beinhalten, daß aufgrund der möglichen Relativbewegungen zwischen handgeführtem Mittel und dem Körper des Patienten diese Zielstrukturen u.U. beschädigt oder verändert werden, besteht in der Technik schon seit längerem der Wunsch, ein Positioniersystem zur Hand zu haben, welches sich am Körper, insbesondere am Schädel mittels einer Halterung ortsfest verankern läßt.

In US-A-4 809 694 wurde eine Vorrichtung zum Halten eines medizinischen Instruments vorgeschlagen, die außen an einem Schädel beispielsweise zwecks Vornahme einer Biopsie von Gehirngewebe befestigt werden kann. Die hier erläuterte Haltevorrichtung weist ein Gehäuse mit einem Außengewinde auf, mittels dessen die Haltevorrichtung in eine zuvor in die Schädeldecke gebohrte Öffnung eingeschraubt wird. In dem Gehäuse ist ein fixierbares Kugelgelenk mit einer die Kugel durchdringenden Bohrung vorgesehen, durch die das zu haltende medizinische Instrument eingeführt wird. Zur Fixierung des medizinischen Instruments innerhalb der die Kugel durchdringenden Bohrung ist die Kugel aus einem gummielastischen Werkstoff hergestellt und weist mittig einen Vorsprung auf, der beim Einführen des medizinischen Instruments deformiert wird und das medizinische Instrument in der Kugel verklemmt. Die in US-A-4 809 694 vorgeschlagene Vorrichtung, die sich ohnehin nicht zur Implantation eignet, ist in mehrfacher Hinsicht nachteilig. So können beispielsweise nur Instrumente eingesetzt werden, deren Außendurchmesser exakt auf die Größe der Bohrung abgestimmt ist, da andernfalls bei zu geringem Außendurchmesser das Instrument nicht ausreichend fixiert wird und bei zu großem Außendurchmesser ein Vorschieben des medizinischen Instruments nicht oder nur unter Schwierigkeiten möglich sein wird. Ferner sind dieser Vorrichtung weder Mittel vorgesehen, um das eingesetzte medizinische Instrument axial zu verstellen, noch lässt sich aufgrund der gummielastischen Eigenschaften der Kugel eine Verlagerung des Zielpunktes des medizinischen Instruments beim Verklemmen der Kugel ausschließen, was sich besonders nachteilig auswirkt, als es zudem nicht möglich ist, die Positionierung des medizinischen Instruments visuell zu verfolgen.

Aus dem Stand der Technik sind insbesondere drei implantierbare Aktorhalterungen bekannt. Eine erste Halterung als Bestandteil eines teilimplantierbaren piezoelektrischen Hörgerätekonzepts zur Stimulation des Steigbügels wurde von N. Yanagihara, K. Gyo und Y. Hinohira in dem in "Otolaryngologic Clinics Of North America" erschienenen Artikel "Partially Implantable Hearing Aid Using Piezoelectric Ceramic Ossicular Vibrator", Vol. 28, No.1, Februar 1995, Seiten 85-97, vorgestellt. Der externe Geräteteil ist wie ein konventionelles, hinter dem Ohr zu tragendes Hörgerät ausgeführt und beinhaltet Mikrophon, Verstärker, Batterie und die externe Sendespule. Der interne, auf der Schädelkalotte fixierte Geräteteil dient zur Aufnahme der inneren Empfangsspule. Zur Positionierung und Fixierung des piezoelektrischen Bimorph-Wandlers im Mittelohr ist ein relativ einfaches L-förmiges knochenverankertes Befestigungselement vorgesehen. Es handelt sich dabei um ein auf der Schädelkalotte mit zwei Knochenschrauben fixierbares Halteblech, das sich aus einer Metallplatte mit zwei Langlöchern und einer senkrecht daran befestigten Drahtachse zusammensetzt. Nach Aufschrauben der Metallplatte auf die Schädelkalotte zeigt die Drahtachse in das Mittelohr (nach medial). Auf der Drahtachse läßt sich eine Hülse axial verschieben und somit der wiederum an der Hülse befestigte piezoelektrische Bimorph-Wandler positionieren. Das ermöglicht einen axialen und einen rotatorischen Freiheitsgrad auf der Drahtachse. Nach Abbau von Hammer und Amboß kann das freie Ende des Piezoelementes vorzugsweise direkt am Steigbügelkopf mit Cyanacrylatkleber befestigt werden.

Ein zweites Haltesystem für ein gehörverbesserndes aktorisches Implantatmodul wurde von J. Frederickson, J.M. Coticchia und S. Khosla in dem ebenfalls in "Otolaryngologic Clinics Of North America" erschienenen Artikel mit dem Titel "Ongoing Investigations Into An Implantable Electromagnetic Hearing Aid For Moderate To Severe Sensorineural Hearing Loss", Vol. 28, No.1., Februar 1995, Seiten 107-119, beschrieben. Es ist Bestandteil eines bisher im Tiermodell erprobten, teilimplantierbaren elektromagnetischen Hörgeräts, bei dessen Implantation mit einem chirurgischen Laser ein kleines Loch in den Amboßkörper zur Befestigung eines Permanentmagneten eingebracht wird. Der Laserkopf wird dabei in einer in den Mastoidknochen eingeschraubten Gewindehülse mit Innen- und Außengewinde geführt, deren Längsachse auf den Amboßkörper zeigt. Nach Setzen der Laserbohrung am Amboß und Entnahme des Laserkopfs kann in diese Gewindehülse der elektromagnetische Antrieb ("transducer probe tip") eingeschraubt und nach medial zum ossikelfesten Magneten positioniert werden.

Ein drittes Haltesystem entwickelten Maniglia et al. für einen teilimplantierbaren elektromagnetischen Mittelohrstimulator [A.J. Maniglia, W.H. Ko, M. Rosenbaum, T. Falk, W.L. Zhu, N.W. Frenz, J. Werning, J. Masin, A. Stein und A. Sabri, "Contactless Semi-Implantable Electromagnetic Middle Ear Device For The Treatment Of Sensorineural Hearing Loss", erschienen in "Otolaryngologic Clinics Of North America", 1995, Vol. 28, No. 1, February 1995, Seiten 121 und folgende]. Dabei wird ein kleiner Magnet mittels chirurgischem Zement auf den Amboß geklebt. Entlang eines aus Titan gefertigten Führungsschaftes, der sich in das Mastoid implantieren läßt, kann die Antriebsspule bis auf einen Luftspalt von maximal 1 mm zum ossikelfesten Permanentmagneten positioniert werden. Dieser Titanschaft besitzt wie in der Ausführung von Yanagihara et al. zwei Langlöcher und ein zusätzliches Bohrloch zur Fixierung mittels dreier Knochenschrauben auf der Schädelkalotte. Mittels einer Gewindeachse lassen sich ein Elektronikmodul und die daran befestigte Antriebsspule nach medial in einer Langlochführung positionieren und über eine Schraube mit Kontermutter auf dem Schaft fixieren.

Alle drei aus dem Stand der Technik bekannten Haltesysteme dienen zur dauerhaften Fixierung von Hörgerätebauteilen auf dem Schädelknochen bzw. in der Nähe des Mittelund Innenohres. Sie zeichnen sich insgesamt durch eine äußerst eingeschränkte intraoperative Positionierbarkeit aufgrund fehlender nutzbarer Freiheitsgrade aus, und sie müssen allesamt durch mehr oder weniger präzises manuelles Zurechtbiegen an die anatomischen Gegebenheiten des Implantationsortes sowie an die vorgefundene Lage des Zielortes im Mittelohr angepaßt werden. Das erste sowie das dritte der beschriebenen Haltesysteme benötigen zudem Kleber bzw. chirurgischen Zement zur Bauteilfixierung, die sich häufig aufgrund von Festigkeitsverlusten nicht als Langzeitimplantate eignen.

So besteht also in mehrerer Hinsicht ein Bedürfnis, eine Vorrichtung zur Hand zu haben, welche ohne Klebstoffe oder chirurgische Zemente dauerhaft am menschlichen Schädel befestigt werden kann, um frei von Relativbewegungen chirurgische, therapeutische oder diagnostische Sensoren bzw. Aktoren im Körper zu positionieren und in der gefundenen Position fest zu fixieren. Die Erfindung stellt sich die Aufgabe, die aus dem Stand der Technik bekannten Nachteile zu vermeiden, d.h. die zu geringe Anzahl der Freiheitsgrade zu erhöhen und somit ein manuelles Zurechtbiegen des Implantathalters zu vermeiden. Zudem soll die räumliche Lage zwischen positioniertem Mittel und körperfestem Zielort sicher und dauerhaft fixierbar sein, damit eine risikobehaftete Positionsänderung des Sensors/Aktors nach erfolgter Implantation sicher vermieden wird.

Diese Aufgabe wird gelöst durch ein dauerhaft implantierbares fixierbares Positioniersystem für die feste, spielfreie Anbindung an den menschlichen Körper, insbesondere an den menschlichen Schädel, mit:
- einer an dem menschlichen Körper fixierbaren Halterung,
- einem an der Halterung angebrachten, mit einem Hilfswerkzeug manuell positionierbaren und durch einen Klemmechanismus fixierbaren Kugelgelenk,
- einer mit der Kugel des Kugelgelenks fest verbundenen Linearantriebsanordnung,
- einem entlang einer Führung der Linearantriebsanordnung geführten Schlitten, der manuell mit einem Hilfswerkzeug über einen Antrieb entlang der Führung frei positionierbar ist, und
- einer an dem Schlitten angebrachten Aufnahme für ein zu positionierendes bzw. fixierendes aktorisches oder sensorisches Mittel.

Das körperfest zu fixierende Positioniersystem dient mit seiner Aufnahme für beliebige aktorische, sensorische, mechanische oder optische Mittel als "künstliche, tremorfreie Hand" des Chirurgen, um das freie Wirkende des Mittels zu einem körperfesten Zielpunkt zu positionieren und dann zu fixieren, ohne daß dabei nennenswerte risikobehaftete Relativbewegungen auftreten.

Bevorzugte Ausführungsformen der Erfindung sind in den Unteransprüchen beschrieben.

Insbesondere kann der Antrieb selbsthemmend ausgeführt sein, so daß unter Anwendung eines möglichst einfachen Aufbaus eines selbsttätige, unbeabsichtigte Verstellung des Schlittens mit dem daran angebrachten Mittel verhindert wird.

Der Antrieb kann insbesondere als Gewindetrieb ausgelegt sein, der eine Gewindespindel sowie ein mit dem Schlitten verbundenes Innengewindeteil aufweist, wobei die Gewindespindel zweckmäßig mit einer Aufnahme für ein Hilfswerkzeug, beispielsweise einen Schraubendreher, versehen ist.

Des weiteren kann der Klemmechanismus zwei Platten aufweisen, zwischen welchen das Kugelgelenk durch Klemmung mittels Klemmschrauben fixierbar ist. Somit lassen sich die drei rotatorischen Freiheitsgrade des aktorischen oder sensorischen Mittels in einfacher Weise fixieren.

In vorteilhafter Weise ist das Positioniersystem so ausgelegt, daß bei gelöster Klemmung des Kugelgelenks für eine Verstellung aller drei rotatorischen Freiheitsgrade desselben nur ein einziges Hilfswerkzeug, wie z.B. ein Inbusschlüssel, erforderlich ist. Zweckmäßigerweise ist das Positioniersystem ferner so ausgelegt, daß die momentane Position der drei rotatorischen Freiheitsgrade bei gelöster Klemmung des Kugelgelenks durch Reibkräfte gesichert ist und nach dem Schließen der Klemmung dauerhaft beibehalten wird.

Um dem behandelnden Operateur das Einbringen und Einstellen des Positioniersystems zu erleichtern, zeigen die Bedienteile zur manuellen Positionierung des Kugelgelenks und des Schlittens sowie zur Klemmung des Kugelgelenks vorzugsweise vom Körper des Patienten weg zum Operateur. In dieser Hinsicht erweist es sich außerdem als zweckmäßig, wenn die Konstruktion und die geometrischen Abmessungen des Positioniersystems derart gestaltet sind, daß der bedienende Operateur stets mit bloßem Auge oder bei Verwendung eines Mikroskopes freie Sicht auf mindestens das freie Wirkende des aktorischen oder sensorischen Mittels sowie auf den Implantationsbereich samt Zielpunkt im Körper des Patienten behält. Auf diese Weise werden die sonst durch eine mögliche Fehlpositionierung des Mittels verursachten Risiken für den Patienten besonders gering gehalten.

Zur Befestigung des Positioniersystems insbesondere an der Oberfläche eines Schädelknochens ist die Halterung vorzugsweise mit Durchbrüchen versehen, in welche Knochenschrauben eingebracht und mit dem Schädelknochen verschraubt werden können. Somit sind für die dauerhafte Fixierung der Vorrichtung am Körper sowie des Mittels in der Vorrichtung keinerlei Klebstoffe oder Knochenzemente notwendig.

Durch die schädelfeste Verschraubung des Positioniersystems werden Relativbewegungen zwischen den im Körper befindlichen, teilweise mikroskopisch kleinen Zielstrukturen und dem im Positioniersystem befestigten Mittel auf ein Minimum reduziert. Bei gelöster Klemmung ist somit eine kontrollierte und risikoarme Positionierung des Mittels und seines Wirkendes relativ zu körperfesten Zielpunkten möglich.

Gemäß einer besonders bevorzugten Ausführungsform handelt es sich bei dem erfindungsgemäßen Positioniersystem um ein manuell bedienbares und in seinen rotatorischen Freiheitsgraden arretierbares Positioniersystem, das die folgenden Bestandteile aufweist:
- eine auf dem Schädelknochen mittels speziellen Knochenschrauben dauerhaft fixierbare Kopfplatte,
- ein an der Kopfplatte angebrachtes, klemmbares Kugelgelenk, beweglich in allen drei rotatorischen Freiheitsgraden,
- eine mit der Kugel des Kugelgelenks fest verbundene Linearantriebsanordnung,
- einen entlang einer Führung der Linearantriebsanordnung geführten Schlitten, der über einen Feingewindetrieb zwischen zwei Endanschlägen der Linearantriebsanordnung frei axial positionierbar ist,
- eine am Schlitten befindliche Aufnahme für das zu positionierende implantierbare Mittel,
- einen Klemmechanismus, der die intraoperativ eingestellte Position aller rotatorischen Freiheitsgrade einschließlich der Lage des Mittels im Schlitten dauerhaft fixiert.

Durch das vorliegend vorgeschlagene klemmbare Positioniersystem, insbesondere durch die nachfolgend erläuterten bevorzugten Ausführungsformen, wird eine Vielzahl von Vorteilen erreicht. Es ergibt sich ein einfacher, kompakter Aufbau aus relativ wenigen Bauteilen, die selbst wiederum einfach aufgebaut und konstruiert sein können. Dies gewährleistet beim bestimmungsgemäßen Gebrauch auch ein hohes Maß an gerätetechnischer Sicherheit für Anwender, Patienten und Dritte. Auch äußere Einflüsse wie z.B. Erschütterungen, Temperatur-, Lage- und Druckwechsel können die Funktion des Systems nicht negativ beeinflussen.

Als bevorzugte Körperregion, in die das Positioniersystem samt Mittel eingebracht werden kann, kommt insbesondere die unter der Ohrmuschel im Schädelknochen liegende Mastoidhöhle in Frage. Sie läßt sich durch mikrochirurgische Standardtechniken eröffnen. Ihr Volumen beträgt dann einige Kubikzentimeter, und dieses Volumen ist großen patientenindividuellen, räumlichen Exemplarstreuungen unterworfen.

Die Kopfplatte des Positioniersystems wird in diesem Fall auf die Oberfläche des unmittelbar an den Rand der geschaffenen Mastoidhöhle angrenzenden Schädelknochens aufgeschraubt. Das System ist so konzipiert, daß es das Niveau der Kalottenwölbung nicht überragt. Somit ist gewährleistet, daß sich das implantierte System nach der Operation nicht unter der Haut abzeichnet.

Mittels des vorliegenden Positioniersystems ist die tremorfreie intraoperative Positionierung und Fixierung eines beliebigen Mittels z.B. an einem der drei Ossikel der Gehörknöchelchenkette (Hammer, Amboß, Steigbügel), der knöchernen Trennwand zwischen luftgefülltem Mittelohr und flüssigkeitsgefülltem Innenohr (Promontorium), im flüssigkeitsgefüllten Innenohr selbst sowie dem angrenzenden Vestibularorgan möglich. Weitere Anwendungen des erfindungsgemäßen Systems liegen in der kurzzeitigen, intraoperativen Laserchirurgie im gesamten Schädelbereich einschließlich Mikrokoagulationen oder Gewebeverödungen. Bei Einkopplung eines Meßlasers können intraoperativ Schwingungen z.B. der Gehörknöchelchenkette, des Trommelfells oder der Rundfenstermembran berührungsfrei gemessen werden.

Bei einer bevorzugten Ausführungsform der Erfindung wird das oben beschriebene implantierbare Positioniersystem mit einem aktorischen Hörgerätewandler kombiniert, der zur gehörverbessernden vibratorischen Stimulation von Schwerhörigen dient. Diese bevorzugte Ausführung ist somit Bestandteil eines teilweise oder vollständig implantierbaren Hörgerätes.

Dabei
- ist die Kopfplatte des Positionier- und Fixiersystems mittels spezieller Knochenschrauben dauerhaft auf dem Schädelknochen fixiert,
- ist in der Aufnahme des Schlittens ein ossikelstimulierender piezoelektrischer Hörgerätewandler befestigt,
- findet das Positioniersystem samt daran befestigtem Hörgerätewandler in der chirurgisch ausgefrästen Mastoidhöhle im Schädelknochen unter der Ohrmuschel Platz, ohne sich postoperativ durch die überdeckende Haut hindurch abzuzeichnen,
- ist der Hörgerätewandler in der Aufnahme des Schlittens so angeordnet, daß sein freies Wirkende in das Körperinnere, bevorzugt in die Räume des Mittelohres zu einem der körperfesten Gehörknöchelchen (Hammer, Amboß oder Steigbügel) zeigt,
- ist das freie Wirkende des Hörgerätewandlers auf einen ausgewählten Zielpunkt der Ossikelkette positionierbar und dort aufsetzbar,
- ist das System so konzipiert, daß verschiedene Zielpunkte der Ossikelkette, wie z.B. Hammergriff (Manubrium), Hammerkörper, Amboßkörper, kurzer Amboßfortsatz, langer Amboßfortsatz, Steigbügelköpfchen oder Steigbügelfußplatte, mit dem freien Wirkende des Hörgerätewandlers erreichbar sind,
- dient bei Ankopplung an den Hammer, Amboßkörper bzw. kurzen Amboßfortsatz der natürlich vorhandene Verbindungskanal (Aditus ad antrum) in der hinteren Gehörgangswand als Durchtritt für das freie Wirkende zwischen Mastoidhöhle und Mittelohr,
- dient bei Ankopplung an den langen Amboßfortsatz, das Steigbügelköpfchen bzw. die Steigbügelfußplatte eine im Chorda-Fazialis-Winkel der hinteren, knöchernen Gehörgangswand geschaffene Bohrung von ca. 2 mm Durchmesser als Durchtritt für das freie Wirkende des Hörgerätewandlers,
- ist das beschriebene erfindungsgemäße Positioniersystem so konzipiert, daß der bedienende Operateur intraoperativ genügend freie Sicht auf das Wirkende des Hörgerätewandlers auch bei Verwendung eines Mikroskopes hat,
- kann der positionierte Hörgerätewandler nach Ankopplung an die Gehörknöchelchenkette durch den beschriebenen erfindungsgemäßen Klemmechanismus dauerhaft und sicher in der intraoperativ definierten Position fixiert werden,
- setzt sich das gesamte Positioniersystem aus langzeitstabilen, biokompatiblen Implantatmaterialien zusammen,
- lassen sich alle Manipulationen zur Befestigung am Schädelknochen, zur Positionierung sowie zur abschließenden Fixierung in situ mit wenigen Hilfswerkzeugen wie z.B. einem Schraubendreher sowie einem Inbusschlüssel bewerkstelligen,
- ist das System so optimiert, daß es zwar die Reactio des schwingenden Piezowandlers aufnimmt, diese aber nicht als vibratorischen Stimulus an den Schädelknochen weiterleitet und somit ein Knochenleitungs-Rücktransport des auditorischen Signals an ein in der Nähe befindliches Mikrophon unterbunden wird,
- ist das Positioniersystem so konzipiert, daß alle seine Bedienelemente auch nach Einbringen in den Implantationsort vom Körper des Patienten weg zeigen, und somit problemlos vom Operateur manuell bedient werden können,
- sind das Ausräumen des Mittelohres sowie das chirurgische Entfernen von Gehörknöchelchen nicht nötig,
- bleiben der äußere Gehörgang gänzlich und die Mittelohrräume weitgehend frei von Implantatkomponenten.

Die oben beschriebenen Merkmale und weitere Merkmale der Erfindung werden nachfolgend unter Bezugnahme auf die beiliegenden Zeichnungen näher erläutert. Es zeigen :
- Fig. 1: eine Schnittansicht durch das implantierbare Positioniersystem nach der Erfindung sowie durch einen Schädelknochen und die Mastoidhöhle;
- Fig. 2: eine schematische Draufsicht auf das in Fig. 1 gezeigte Positioniersystem;
- Fig. 3: eine Schnittansicht der Mastoidhöhle und des Mittelohres sowie des Positioniersystems nach der Erfindung, wobei die Kopfplatte auf der Schädeloberfläche fixiert ist, die Linearantriebsanordnung um das Kugelzentrum maximal geschwenkt ist und das freie Wirkende eines Hörgeräteaktors durch die hintere Gehörgangswand zum Zielpunkt im Mittelohr zeigt; und
- Fig. 4: eine schematische Schnittdarstellung des Mittelohres mit möglichen Ankoppelpunkten für das freie Wirkende des Hörgeräteaktors.

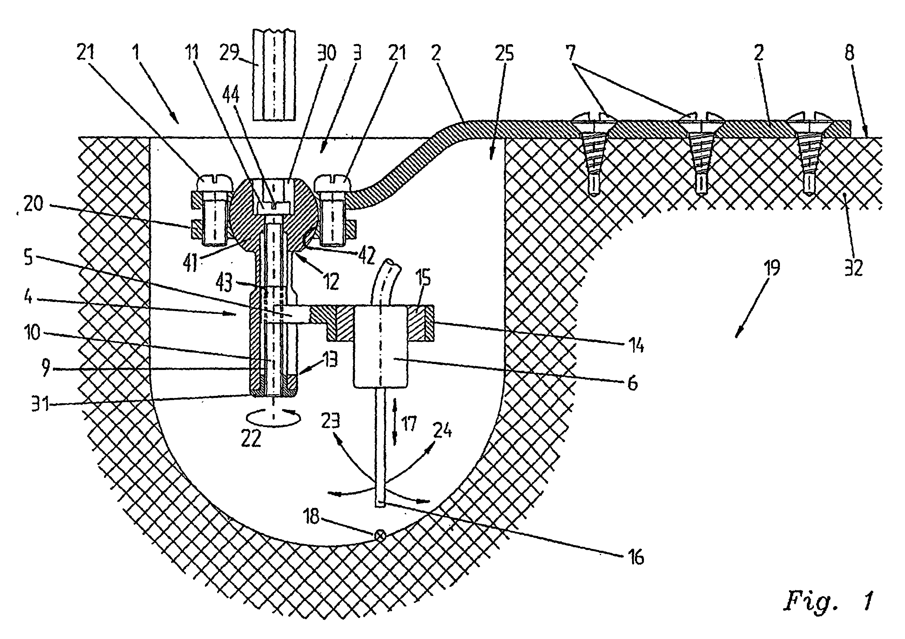

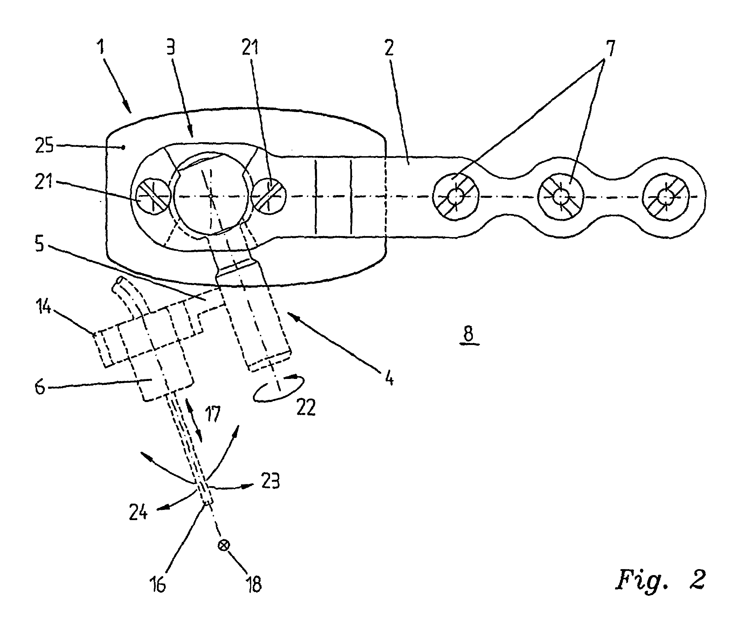

Das in den Figuren 1 und 2 dargestellte implantierbare Positionier- und Fixiersystem 1 weist eine zur Knochenverankerung geeignete Kopfplatte 2, ein klemmbares Kugelgelenk 3, eine daran fest fixierte Linearantriebsanordnung 4 sowie einen entlang der Linearantriebsanordnung geführten Schlitten 5 zur Aufnahme eines beliebigen aktorischen oder sensorischen Mittels 6 auf. Zu dem klemmbaren Kugelgelenk 3 gehören eine Gelenkkugel 41 und ein insgesamt mit 42 bezeichneter Kugelsitz.

Die Kopfplatte 2 des Gesamtsystems 1 läßt sich durch Knochenschrauben 7 auf Knochenoberflächen 8, wie z.B. dem Schädelknochen 32, festschrauben. Das freie Ende der Kopfplatte ist stufig abgesetzt. Daran sind das klemmbare Kugelgelenk 3, die Linearantriebsanordnung 4 sowie das im Schlitten 5 aufgenommene Mittel 6 angebracht.

Durch das stufige Absetzen des freien Endes der Kopfplatte 2 wird nach dem Aufschrauben der Kopfplatte auf die Knochenoberfläche 8 des Schädels 32 und dem damit verbundenen Einbringen der Systemhauptkomponenten (Kopfplatte 2, Kugelgelenk 3, Linearantriebsanordnung 4, Schlitten 5 und Mittel 6) in eine geeignete Körperhöhle 25 ein Überragen des Positioniersystems 1 über das Niveau der Knochenoberfläche 8 vermieden.

Die Kugel 41 des klemmbaren Kugelgelenks 3 wird in entsprechenden Kugelsenkungen zwischen der Kopfplatte 2 und einer davon in Abstand mindestens näherungsweise parallel angeordneten Konterplatte 20 zentriert und gehalten. Die beiden Platten 2 und 20 bilden dabei gemeinsam den Kugelsitz 42, und sie lassen sich durch eine oder mehrere Klemmschrauben 21 zusammen- bzw. auseinanderbewegen. Es versteht sich jedoch, daß der Kugelsitz des klemmbaren Kugelgelenks auch als von der Kopfplatte 2 gesonderte Baugruppe ausgebildet sein kann, die an der Kopfplatte befestigt ist.

Durch Anziehen der Klemmschrauben 21 wird die Kugel 41 fest in die Kugelsenkungen des Kugelsitzes 42 gepreßt. Die momentane Position der Gelenkkugel 41, der an der Gelenkkugel befestigten Linearantriebsanordnung 4 sowie des mit der Linearantriebsanordnung in Antriebsverbindung stehenden aktorischen oder sensorischen Mittels 6 wird somit auf einfache Weise räumlich fixiert.

Durch Lösen der Klemmschrauben 21 ist die Kugel 41 des Kugelgelenks 3 in ihrem Kugelsitz 42 in allen drei rotatorischen Freiheitsgraden 22, 23, 24 frei schwenkbar. Dabei ist eine vollständige 360°-Drehung (Pfeil 22) um die Längsachse der Linearantriebsanordnung 4 möglich. Die Schwenkwinkel entlang der beiden weiteren rotatorischen Freiheitsgrade 23 und 24 betragen bei dem veranschaulichten Ausführungsbeispiel jeweils ungefähr 160°.

Zum Positionieren des Kugelgelenks 3 werden die Klemmschrauben 21 nur soweit gelöst, daß die Kugel 41 durch Reibkräfte in den Kugelsenkungen von Kopfplatte 2 und Konterplatte 20 noch gehalten wird. Eine Positionsänderung der momentanen Lage des Mittels 6 z.B. durch Verkipppen aufgrund der wirkenden Gravitationskräfte wird somit vermieden.

Auf der körperabgewandten, also zum bedienenden Operateur zeigenden Seite der Kugel 41 ist eine Vertiefung 30 eingebracht, in die ein Hilfswerkzeug 29, wie z.B. ein Inbusschlüssel, formschlüssig eingesteckt werden kann. Damit läßt sich die Kugel 41 des Kugelgelenks 3 in allen drei rotatorischen Freiheitsgraden 22, 23, 24 im Kugelsitz 42 drehen.

Die Linearantriebsanordnung 4 weist eine geradlinige Führung (Führungsschiene) 9 auf, entlang welcher der Schlitten 5 spielfrei geführt ist. Zum Verfahren des Schlittens 5 ist ein in die Linearantriebsanordnung 4 integrierter Antrieb vorgesehen, zu dem bei der gezeigten Ausführungsform eine Gewindespindel 10 und ein damit in Gewindeeingriff stehendes, mit dem Schlitten 5 verbundenes Innengewindeteil 43 gehören. Das Innengewindeteil 43 bildet zweckmäßig, wie veranschaulicht, einen Bestandteil des Schlittens 5, und die Gewindespindel 10 weist ein Bedienende 11 auf, welches mit der Gewindespindel 10 fest verbunden ist. Die Gewindesteigungen der Gewindespindel 10 und des Innengewindeteils 43 sind selbsthemmend ausgelegt, das heißt, ein Verstellen des Schlittens 5 ist nur durch Drehen der Gewindespindel 10 möglich, während von dem Schlitten 5 auf die Gewindespindel 10 übertragene Kräfte nicht in der Lage sind, die Gewindespindel 10 zu drehen. Infolgedessen ist ausgeschlossen, daß sich der Schlitten 5 entlang der Linearführung 9 aufgrund von z.B. Gravitationskräften ungewollt selbsttätig bewegen kann.

Durch formschlüssiges Einsetzen eines Hilfswerkzeuges, beispielsweise eines Schraubendrehers, in eine Vertiefung 44 am Bedienende 11 der Gewindespindel 10 und durch manuelle Drehbewegung am Bedienende 11 wird entsprechend dem Gewindedrehsinn und der gewählten Gewindesteigung von Gewindespindel 10 und Innengewindeteil 43 eine Axialverschiebung des Schlittens 5 entlang der Führung 9 der Linearantriebsanordnung 4 bewirkt. Am kugelgelenkseitigen Ende der Linearantriebsanordnung 4 ist der Axialweg des Schlittens 5 durch einen an der Kugel 41 des klemmbaren Kugelgelenks 3 ausgebildeten Endanschlag 12 begrenzt. Am gegenüberliegenden Ende der Linearantriebsanordnung dient ein mit der Führung 9 fest verbundener Endanschlag 13 als Wegbegrenzung. Alternativ zu dem Endanschlag 13 kann ein mit der Gewindespindel 10 fest verbundener, drehbarer Abschlußdeckel 31 als Endanschlag zum Begrenzen des Axialweges des Schlittens 5 genutzt sein. Der Schlitten 5 läßt sich somit entlang der Linearantriebsanordnung 4 zwischen den Endanschlägen 12 und 13 bzw. 31 stufenlos verfahren, und er hält aufgrund der Selbsthemmung der Gewindetriebs 10, 11 seine momentane Position. Der Verfahrweg des Schlittens 5 auf der Linearantriebsanordnung 4 beträgt bei einer bevorzugten Ausführungsform 5 bis 10 mm. In den Figuren 1 und 2 ist der Schlitten 5 in Mittelstellung zwischen den beiden Endanschlägen dargestellt.

Der Schlitten 5 besitzt eine Aufnahme 14, in die das gewünschte aktorische oder sensorische Mittel 6 spielfrei eingesetzt werden kann. Bei der veranschaulichten Ausführungsform hat letzteres eine Längsachse, die mindestens näherungsweise parallel zu der Längsachse der Gewindespindel 10 und der Führung 9 verläuft, gegenüber dieser aber in Querrichtung versetzt ist. Falls eine mechanische Entkoppelung oder eine elastische Lagerung zwischen Positioniersystem 1 und dem in der Aufnahme 14 fixierten Mittel 6 erforderlich ist, kann zwischen Aufnahme 14 und Mittel 6 ein elastisches bzw. federelastisches Zwischenstück 15 eingefügt sein.

Das freie Wirkende 16 des in die Aufnahme 14 des Schlittens 5 eingelegten Mittels 6 ist somit durch Drehen des Bedienendes 11 der Gewindespindel 10 parallel zu der Linearführung 9 in axialer Richtung 17 mit Bezug auf einen körperfesten Zielpunkt 18 im menschlichen Körper 19 positionierbar.

Bei gelöster Klemmung des Kugelgelenks 3 läßt sich die gesamte Linearantriebsanordnung 4 um das Zentrum der ortsfest zwischen Kopfplatte 2 und Konterplatte 20 gelagerten Kugel 41 in allen drei rotatorischen Freiheitsgraden des Raums 22, 23, 24 drehen. In Verbindung mit dem axialen Freiheitsgrad 17 der Linearantriebsanordnung 4 ergeben sich somit vier für die Positionierung des Mittels 6 verfügbare Freiheitsgrade im Raum.

In Figur 2 ist dargestellt, daß mit dem Positioniersystem 1 auch unter der Schädelkalotte gelegene Zielpunkte 18 durch das freie Wirkende 16 des im System befestigten Mittels 6 erreichbar sind.

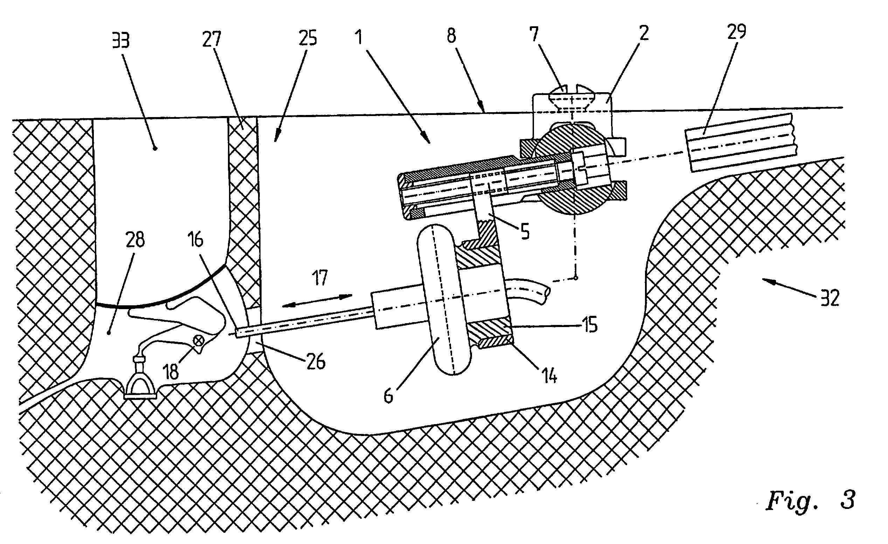

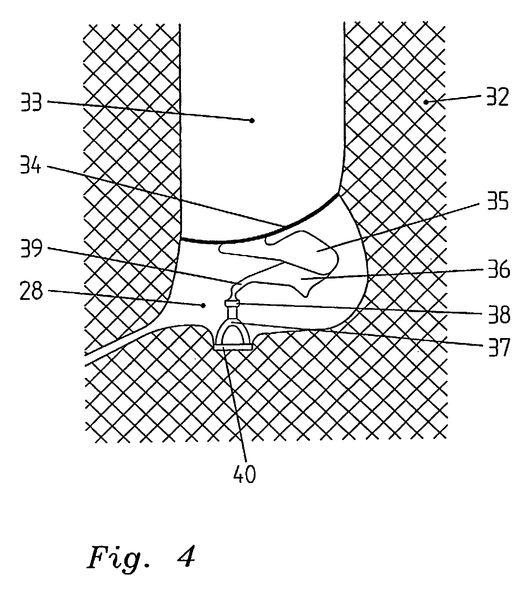

In Figur 3 ist eine bevorzugte Ausführung des Positionier- und Fixiersystems dargestellt. Figur 4 zeigt mögliche Ankoppelpunkte im Mittelohr 28, das vom äußeren Gehörgang 33 durch das Trommelfell 34 abgegrenzt ist. Als Aktor 6 wird bei der Anordnung nach Fig. 3 ein zur vibratorischen Stimulierung der Ossikelkette geeigneter Hörgerätewandler in der Aufnahme 14 des Schlittens 5 gehalten. Der Hörgerätewandler ist Bestandteil eines teilweise oder vollständig implantierbaren Hörgerätes. Bei dieser bevorzugten Ausführung ist der körperfeste Zielpunkt 18 ein Punkt auf der Ossikelkette, wahlweise Hammer 35, Amboß 36 oder Steigbügel 37 (FIG. 4).

Die Körperöffnung 25, in die das Positioniersystem 1 gemäß dieser bevorzugten Ausführungsform implantiert und intraoperativ mit Hilfswerkzeugen positioniert und fixiert werden kann, ist die Mastoidhöhle 25 im Schädelknochen 32. Die Kopfplatte 2 ist auf die Oberfläche 8 des an die Mastoidhöhle angrenzenden Schädelknochens 32 aufgeschraubt. Das freie Wirkende 16 des aktorischen Hörgerätewandlers 6 reicht durch einen Knochendurchbruch 26 der hinteren Gehörgangswand 27 in das Mittelohr 28. Je nach anatomisch vorgefundener Situation von Mastoidhöhle 25, hinterer Gehörgangswand 27 und Mittelohr 28 entscheidet der Operateur über den jeweils am besten geeigneten Zielpunkt 18 auf der Ossikelkette (Hammer 35, Amboß 36, Steigbügel 37).

Zur Ankopplung an den Amboßkörper 36 kann als Knochendurchbruch 26 für das freie Wirkende 16 des Hörgerätewandlers 6 ein natürlich vorhandener Kanal in der hinteren Gehörgangswand 27, der Aditus ad antrum, genutzt werden. Für die Ankopplung an den langen Amboßfortsatz 39, den Processus lenticularis 38 sowie an Strukturen des Steigbügels 37, z.B. die Steigbügelplatte 40, muß ein geeigneter Knochendurchbruch 26 in der hinteren Gehörgangswand 27 gebohrt werden. Dieser Durchbruch wird im Chorda-Fazialis-Winkel geschaffen und besitzt zweckmäßig einen Durchmesser von etwa 2 mm.

Die Ankopplung des freien Wirkendes 16 des Hörgerätewandlers 6 an die Gehörknöchelchen des Mittelohres (Hammer, Amboß, Steigbügel) sowie an Strukturen des Innenohres und des Vestibularorgans kann in beliebiger bekannter Weise erfolgen.

Geeignete aktorische bzw. sensorische Mittel 6 für die Anwendung des Positioniersystems 1 sind unter anderem aktive elektromechanische Hörgerätewandler zur elektromechanischen Stimulation der Gehörknöchelchenkette, Erregerspulen zur elektromagnetischen Stimulation von ossikelkettenfest fixierten Permanentmagneten, Lichtleiter zur Führung von chirurgischem Laserlicht (z.B. zum Schneiden, Bohren, Koagulieren oder Veröden von Gewebe oder Knochenstrukturen), Lichtleiter zur Führung von meßtechnischem Laserlicht (Laser-Doppler-Vibrometrie), flexible Miniaturendoskope zur Inspektion beliebiger Schädelregionen, Sondenmikrophone und kleine Schallquellen zur intraoperativen Audiometrie (Hörschwellenbestimmung, Ableitung von otoakustischen Emissionen) sowie Elektroden zur Ableitung von elektrocochleographischen Körperpotentialen (z.B. Summenaktionspotential oder Mikrophonpotential) bzw. zur Elektrostimulation im Rahmen präoperativer Hörtests vor Implantation von Cochlear Implants (Promontorialtest).

## Patentansprüche

1. Dauerhaft implantierbares fixierbares Positioniersystem für die feste, spielfreie Anbindung an den menschlichen Körper, insbesondere an den menschlichen Schädel, mit:
- einer an dem menschlichen Körper fixierbaren Halterung (2),
- einem an der Halterung (2) angebrachten, mit einem Hilfswerkzeug manuell positionierbaren und durch einen Klemmechanismus (2, 20, 21) fixierbaren Kugelgelenk (3),
- einer mit der Kugel (41) des Kugelgelenks (3) fest verbundenen Linearantriebsanordnung (4),
- einem entlang einer Führung (9) der Linearantriebsanordnung (4) geführten Schlitten (5), der manuell mit einem Hilfswerkzeug über einen Antrieb (10, 11) entlang der Führung frei positionierbar ist, und
- einer an dem Schlitten (5) angebrachten Aufnahme (14) für ein zu positionierendes bzw. fixierendes aktorisches oder sensorisches Mittel (6).

2. Positioniersystem nach Anspruch 1, **dadurch gekennzeichnet, daß** der Antrieb (10, 11) selbsthemmend ausgeführt ist.

3. Positioniersystem nach Anspruch 1 oder 2, **dadurch gekennzeichnet, daß** das Positioniersystem aufgrund der Freiheitsgrade der Linearantriebsanordnung (4) und des Kugelgelenks (3) zusammengefaßt für eine axiale (17) und drei rotatorische (22, 23, 24) Bewegungen des aktorischen oder sensorischen Mittels (6) und seines freien Wirkendes (16) in Körperöffnungen (25) des menschlichen Körpers (19) ausgelegt ist.

4. Positioniersystem nach einem der vorhergehenden Ansprüche, **dadurch gekennzeichnet, daß** der Antrieb als Gewindetrieb (10, 11) ausgelegt ist.

5. Positioniersystem nach Anspruch 4, **dadurch gekennzeichnet, daß** der Gewindetrieb (10, 11) eine Gewindespindel (10) mit einer Aufnahme (44) für ein Hilfswerkzeug sowie ein mit dem Schlitten (5) verbundenes Innengewindeteil (43) aufweist.

6. Positioniersystem nach einem der vorhergehenden Ansprüche, **dadurch gekennzeichnet, daß** der Klemmechanismus zwei Platten (2, 20) aufweist, zwischen welchen das Kugelgelenk (3) durch Klemmung mittels Klemmschrauben (21) fixierbar ist.

7. Positioniersystem nach Anspruch 6, **dadurch gekennzeichnet, daß** es bei gelöster Klemmung des Kugelgelenks (3) für eine Verstellung aller drei rotatorischen Freiheitsgrade (22, 23, 24) desselben mittels eines einzigen Hilfswerkzeugs (28) ausgelegt ist.

8. Positioniersystem nach einem der vorhergehenden Ansprüche, **dadurch gekennzeichnet, daß** die momentane Position der drei rotatorischen Freiheitsgrade (22, 23, 24) bei gelöster Klemmung des Kugelgelenks (3) durch Reibkräfte gesichert ist und nach dem Schließen der Klemmung (2, 20, 21) dauerhaft beibehalten wird.

9. Positioniersystem nach einem der vorhergehenden Ansprüche, **dadurch gekennzeichnet, daß** die Halterung (2) mit Durchbrüchen für das Einbringen von Knochenschrauben (7) zum Aufschrauben des Positioniersystems auf die Oberfläche (8) eines Schädelknochens (32) versehen ist.

10. Positioniersystem nach einem der vorhergehenden Ansprüche, **dadurch gekennzeichnet, daß** das Kugelgelenk (3) eine Aufnahme (30) für ein Hilfswerkzeug (29) aufweist.

11. Positioniersystem nach einem der vorhergehenden Ansprüche, **dadurch gekennzeichnet, daß** die Bedienteile (21, 30, 44) zur manuellen Positionierung des Kugelgelenks (3) und des Schlittens (5) sowie zur Klemmung des Kugelgelenks (3) vom Körper des Patienten weg zum Operateur zeigen.

12. Positioniersystem nach einem der vorhergehenden Ansprüche, **dadurch gekennzeichnet, daß** die Konstruktion und die geometrischen Abmessungen des Positioniersystems derart gestaltet sind, daß der bedienende Operateur sowohl bei Arbeiten mit bloßem Auge als auch bei Verwendung eines Mikroskopes freie Sicht auf mindestens das freie Wirkende (16) des aktorischen oder sensorischen Mittels (6) sowie den Implantationsbereich samt Zielpunkt (18) im Körper (19) des Patienten hat.

13. Positioniersystem nach einem der vorhergehenden Ansprüche, **dadurch gekennzeichnet, daß** es aus implantierbaren Metallen wie z.B. Reintitan, seinen implantierfähigen Legierungen sowie Implantat-Edelstählen gefertigt ist.

14. Positioniersystem nach einem der vorhergehenden Ansprüche, **dadurch gekennzeichnet, daß** die aktorischen oder sensorischen Mittel (6) zur Diagnose, Therapie und/oder für chirurgische Applikationen für die temporäre oder dauerhafte Implantation der genannten Mittel ausgelegt sind.

15. Positioniersystem nach einem der vorhergehenden Ansprüche, **dadurch gekennzeichnet, daß** es sich bei dem aktorischen Mittel (6) um einen implantierbaren elektromechanischen Hörgerätewandler handelt.

16. Positioniersystem nach einem der vorhergehenden Ansprüche, **dadurch gekennzeichnet, daß** es zum Positionieren und Ankoppeln eines daran befestigten Hörgerätewandlers (6) zu Hammer (35), Amboß (36) oder Steigbügel (37) als körperfestem Zielpunkt (18) und zum Fixieren des Hörgerätewandlers in dieser Lage ausgelegt ist.

17. Positioniersystem nach Anspruch 16, **dadurch gekennzeichnet, daß** der Hörgerätewandler (6) als aktorische Komponente eines teilweise oder vollständig implantierbaren Hörgerätes vorgesehen ist.

18. Positioniersystem nach einem der vorhergehenden Ansprüche, **dadurch gekennzeichnet, daß** die Linearachsanordnung (4) mit Endanschlägen (12, 13, 31) versehen ist, zwischen denen der Schlitten (5) entlang der Führung (9) verstellbar ist.

## Claims

1. Permanently implantable, fixable positioning system for fixed, play-free connection to the human body, in particular to the human cranium, having:
■ a mount (2) fixable to the human body,
■ a ball joint (3) which is mounted on the mount (2), is manually positionable with an auxiliary tool, and is fixable by a clamping mechanism (2, 20, 21),
■ a linear drive arrangement (4) rigidly connected to the ball (41) of the ball joint (3),
■ a slide (5) guided along a guide (9) of the linear drive arrangement (4), which slide is freely positionable along the guide manually with an auxiliary tool via a drive (10, 11), and
■ a seat (14) mounted on the slide (5) for an actoric or sensoric means (6) to be positioned or fixed.

2. Positioning system according to claim 1, **characterised in that** the drive (10, 11) is self-locking.

3. Positioning system according to claim 1 or 2, **characterised in that** due to the degree of freedom of the linear drive arrangement (4) and of the ball joint (3), the positioning system is wired together for an axial (17) and three rotary (22, 23, 24) movements of the actoric or sensoric means (6) and of its free operating end (16) in apertures (25) of the human body (19).

4. Positioning system according to one of the preceding claims, **characterised in that** the drive is wired as a threaded drive (10, 11).

5. Positioning system according to claim 4, **characterised in that** the threaded drive (10, 11) has a threaded spindle (10) with a seat (44) for an auxiliary tool and a female-threaded part (43) connected to the slide (5).

6. Positioning system according to one of the preceding claims, **characterised in that** the clamping mechanism has two plates (2, 20), between which the ball joint (3) is fixable by clamping with clamping screws (21).

7. Positioning system according to claim 6, **characterised in that** when the ball joint (3) is released from clamping, the system is wired for adjustment of all three rotary degrees of freedom (22, 23, 24) thereof by means of a single auxiliary tool (28).

8. Positioning system according to one of the preceding claims, **characterised in that** the momentary position of the three rotary degrees of freedom (22, 23, 24), when the ball joint (3) is released from clamping, is secured by friction forces and is retained permanently after closing of the clamp (2, 20, 21).

9. Positioning system according to one of the preceding claims, **characterised in that** the mount (2) is provided with perforations for the insertion of bone screws (7) for screwing the positioning system on to the surface (8) of a cranial bone (32).

10. Positioning system according to one of the preceding claims, **characterised in that** the ball joint (3) has a seat (30) for an auxiliary tool (29).

11. Positioning system according to one of the preceding claims, **characterised in that** the operating parts (21, 30, 44) for manual positioning of the ball joint (3) and of the slide (5) as well as for clamping the ball joint (3) point away from the body of the patient towards the operator.

12. Positioning system according to one of the preceding claims, **characterised in that** the construction and geometric dimensions of the positioning system are so formed that the operator has a clear view of at least the free operating end (16) of the actoric or sensoric means (6) as well as the implantation area, together with the destination (18) in the patient's body (19), whether working with the naked eye or using a microscope.

13. Positioning system according to one of the preceding claims, **characterised in that** it is manufactured from implantable metals, such as pure titanium, its implantable alloys, or implantable high-quality steels.

14. Positioning system according to one of the preceding claims, **characterised in that** the actoric or sensoric means (6) are wired for diagnosis, treatment, and/or for surgical application for the temporary or permanent implantation of the said means.

15. Positioning system according to one of the preceding claims, **characterised in that** the actoric means (6) are an implantable, electromechanical hearing-aid transformer.

16. Positioning system according to one of the preceding claims, **characterised in that** it is wired for positioning and coupling of an attached hearing-aid transformer (6) to the malleus (35), the incus (36), or the stapes (37) as the destination (18) in the body and for fixing of the hearing-aid transformer in this position.

17. Positioning system according to claim 16, **characterised in that** the hearing-aid transformer (6) is provided as the actoric component of a partially or completely implantable hearing aid.

18. Positioning system according to one of the preceding claims, **characterised in that** the linear drive arrangement (4) is provided with limit stops (12, 13, 21), between which the slide (5) is adjustable along the guide (9).

## Revendications

1. Système durablement implantable de positionnement pouvant être fixé pour être attaché de manière fixe et sans jeu sur le corps humain, notamment sur le crâne humain, comprenant :
- un support (2) pouvant être fixé sur le corps humain,
- une articulation à rotule (3) disposée sur le support (2) pouvant être positionnée manuellement à l'aide d'un outil auxiliaire et pouvant être fixée à l'aide d'un mécanisme de serrage (2, 20, 21),
- un ensemble d'entraînement linéaire (4) rigidement lié à la bille (41) de la rotule (3),
- un chariot (5) guidé le long d'un guidage (9) de l'ensemble d'entraînement linéaire (4), qui peut être positionné librement le long du guidage manuellement à l'aide d'un outil auxiliaire par l'intermédiaire d'un entraînement (10, 11), et
- un logement (14) prévu sur le chariot (5) pour un moyen actorique ou sensoriel (6) à positionner ou à fixer.

2. Système de positionnement selon la revendication 1, **caractérisé en ce que** l'entraînement (10, 11) est réalisé à blocage automatique.

3. Système de positionnement selon la revendication 1 ou 2, **caractérisé en ce que** le système de positionnement, en raison des degrés de liberté de l'ensemble d'entraînement linéaire (4) et de la rotule (3), est conçu pour un mouvement axial (17) et trois mouvements de rotation (22, 23, 24) du moyen actorique ou sensoriel (6) et de son extrémité active libre (16) dans des orifices (25) du corps humain (19).

4. Système de positionnement selon l'une quelconque des revendications précédentes, **caractérisé en ce que** l'entraînement est un entraînement à filet (10, 11).

5. Système de positionnement selon la revendication 4, **caractérisé en ce que** l'entraînement à filet (10, 11) présente une tige filetée (10) avec un logement (44) pour un outil auxiliaire ainsi qu'un taraudage (43) solidaire du chariot (5).

6. Système de positionnement selon l'une quelconque des revendications précédentes, **caractérisé en ce que** le mécanisme de serrage présente deux plaques (2, 20) entre lesquelles la rotule (3) peut être fixée par serrage à l'aide de vis de serrage (21).

7. Système de positionnement selon la revendication 6, **caractérisé en ce qu'**il permet, la rotule (3) étant desserrée, un ajustement des trois degrés de liberté de rotation (22, 23, 24) de cette dernière à l'aide d'un seul outil auxiliaire (28).

8. Système de positionnement selon l'une quelconque des revendications précédentes, **caractérisé en ce que** la position momentanée des trois degrés de liberté de rotation (22, 23, 24), la rotule (3) étant desserrée, est assurée par des forces de friction et maintenue durablement après la fermeture du serrage (2, 20, 21).

9. Système de positionnement selon l'une quelconque des revendications précédentes, **caractérisé en ce que** le support (2) est pourvu de passages pour l'introduction de vis d'os (7) pour visser le système de positionnement sur la surface (8) d'un os crânien (32).

10. Système de positionnement selon l'une quelconque des revendications précédentes, **caractérisé en ce que** la rotule (3) présente un logement (30) pour un outil auxiliaire (29).

11. Système de positionnement selon l'une quelconque des revendications précédentes, **caractérisé en ce que** les éléments de manipulation (21, 30, 44) pour le positionnement manuel de la rotule (3) et du chariot (5) ainsi que pour le serrage de la rotule (3) sont orientés depuis le corps du patient vers l'opérateur.

12. Système de positionnement selon l'une quelconque des revendications précédentes, **caractérisé en ce que** la construction et les dimensions géométriques du système de positionnement sont prévues de telle sorte que l'opérateur, aussi bien en travaillant à l'oeil nu qu'en utilisant un microscope, a une vue dégagée au moins sur l'extrémité active libre (16) du moyen actorique ou sensoriel (6) ainsi que sur la zone d'implantation et le point cible (18) dans le corps (19) du patient.

13. Système de positionnement selon l'une quelconque des revendications précédentes, **caractérisé en ce qu'**il est réalisé à partir de métaux implantables tels que le titane pur, les alliages implantables de celui-ci ainsi qu'à partir d'aciers spéciaux à implants.

14. Système de positionnement selon l'une quelconque des revendications précédentes, **caractérisé en ce que** le. moyen actorique ou sensoriel (6) est conçu pour le diagnostic, la thérapie et/ou pour des applications chirurgicales pour l'implantation temporaire ou durable desdits moyens.

15. Système de positionnement selon l'une quelconque des revendications précédentes, **caractérisé en ce que** le moyen actorique (6) est un transducteur de prothèse auditive électromécanique implantable.

16. Système de positionnement selon l'une quelconque des revendications précédentes, **caractérisé en ce qu'**il est conçu pour positionner et accoupler un transducteur de prothèse auditive (6) fixé sur lui par rapport au marteau (35), à l'enclume (36) ou à l'étrier (37) en tant que point cible (18) du corps, et pour fixer le transducteur de prothèse auditive dans cette position.

17. Système de positionnement selon la revendication 16, **caractérisé en ce que** le transducteur de prothèse auditive (6) est prévu comme composant actorique d'une prothèse auditive partiellement ou complètement implantable.

18. Système de positionnement selon l'une quelconque des revendications précédentes, **caractérisé en ce que** l'ensemble d'entraînement linéaire (4) est pourvu de butées d'extrémité (12, 13, 31) entre lesquelles le chariot (5) est réglable le long du guidage (9).
